# EUROPEAN PATENT APPLICATION

(11) **EP 2 762 080 A1**
(43) Date of publication of application: **06.08.2014**
(21) Application number: 12835677.1
(22) Date of filing: 28.09.2012
(51) Int. Cl.: A61B 6/00, A61B 6/02

(54) **RADIOGRAPH DISPLAY METHOD AND EQUIPMENT**

(30) Priority: 30.09.2011 US 201161541270 P
(71) Applicant: FUJIFILM Corporation, Tokyo 106-8620 (JP)
(72) Inventor: KUWABARA, Takao, Ashigarakami-gun Kanagawa 258-8538 (JP); YAHIRO, Yasuko, Ashigarakami-gun Kanagawa 258-8538 (JP); OHTA, Yasunori, Ashigarakami-gun Kanagawa 258-8538 (JP); HASEGAWA, Akira, San Jose, California 95131 (US)
(74) Representative: Klunker . Schmitt-Nilson . Hirsch
(86) International application number: PCT/JP2012/006231
(87) International publication number: WO 2013/046709

(57) **Abstract**

[Objective]

To enable instantaneous confirmation regarding whether targeting of a lesion by a stereobiopsy device or the like has been performed correctly and improve diagnostic efficiency.

[Constitution]

Two radiation images are acquired by emitting radiation to a subject from two imaging directions that form a first imaging angle, and two radiation images are acquired imaged from two imaging directions that form a second imaging angle which differs from the first imaging angle. The radiation images of the first imaging angle are displayed as two-dimensional images while the radiation images of the second imaging angle are displayed as a stereoscopic image. Designation of a predetermined position in the radiation images of the first imaging angle that are displayed as two-dimensional images is received to obtain position information. The obtained position information in the radiation images of the first imaging angle is projected onto the radiation images of the second imaging angle based on the position information and three-dimensional position information is obtained. Based on the three-dimension position information, markers are displayed on the radiation images of the second imaging angle which are displayed as a stereoscopic image.

## Description

### BACKGROUND OF THE INVENTION

### Field of the Invention

The present invention relates generally to a radiation image display method and a radiation image display apparatus which are applied to a stereo biopsy apparatus which identifies a position of a lesion or the like by using radiation images having imaging directions that differ from each other.

### Description of the Related Art

Pieces of tissues are sometimes collected from around lesions in clinical examinations. Recently, as a method for obtaining pieces of tissues without placing heavy burden on patients, biopsy, in which a hollow tissue collection needle (hereinafter, referred to as a "biopsy needle") is inserted into a patient and tissue that fills the hollow space of the needle is collected, has received wide attention. Further, a stereo biopsy apparatus has been proposed as an apparatus for implementing such biopsy (for example, refer to Patent Document 1).

The stereo biopsy apparatus is an apparatus that emits radiation onto a subject from different directions to obtain a plurality of radiation images and that displays these radiation images as a stereoscopic image.

Then, targeting is performed on the displayed plurality of two-dimensional images so as to obtain position information regarding each of the targets, and three-dimensional position information of the position of a lesion is calculated based on the obtained position information. Further, the tip of a biopsy needle is controlled to achieve the three-dimensional position thereof so that pieces of tissues are collected from a desired position.

### [Prior Art Documents]

### [Patent Documents]

Patent Document 1: PCT Japanese Publication No. 2000-500031

### SUMMARY OF THE INVENTION

However, if targeting is performed on two radiation images which are two-dimensionally displayed separately as described above, it is difficult to target the same lesion in each of the two radiation images. Specifically, in the case that a great numbers of calcifications exist in radiation images of a breast, it is difficult to choose calcifications which properly correspond to each other in two radiation images. Moreover, if calcifications which are different in right and left images, a biopsy needle is likely to be punctured into a wrong position. For such reasons, conventional stereo biopsy apparatuses require a half to one hour to perform tissue collection, which causes deterioration in diagnostic efficiency.

In view of the above circumstance, it is an object of the present invention to provide a radiation image display method and apparatus that enable instantaneous confirmation regarding whether targeting of a lesion by a stereobiopsy device or the like has been performed correctly and that improve diagnostic efficiency.

A radiation image display apparatus of the present invention includes:
a radiation image acquisition section which acquires two radiation images imaged by emitting radiation to a subject from two imaging directions that form a first imaging angle and which acquires two radiation images of the subject from two imaging directions that form a second imaging angle which differs from the first imaging angle;
a display section which displays the radiation images of the first imaging angle as two-dimensional images and which displays the radiation images of the second imaging angle a stereoscopic image;
a position designation receiving section which receives designation of a predetermined position within the radiation images of the first imaging angle which are displayed as the two-dimensional images;
a first position information obtainment section which obtains position information received by the position designation receiving section; and
a second position information obtainment section which obtains three-dimensional position information by projecting the position information within the radiation images of the first imaging angle, that has been obtained by the first position information obtainment section, onto the radiation images of the second imaging angle based on the obtained position information; wherein
the display section displays three-dimensional markers on the radiation images of the second imaging angle, which are displayed as the stereoscopic image, based on the three-dimensional position information obtained by the second position information obtainment section.

In the radiation image display apparatus of the present invention as described above, the above first imaging angle may be larger than the above second imaging angle.

Further, images imaged by emitting radiation to the subject from the two imaging directions that form the second imaging angle as described above may be used as the radiation images of the second imaging angle.

Further, an image conversion section which generates radiation images of the second imaging angle by using the radiation images of the first imaging angle may be provided, and the radiation image acquisition section may acquire radiation images of the second imaging angle generated by the image conversion section.

A radiation image display method of the present invention includes the steps of:
acquiring two radiation images imaged by emitting radiation to a subject from two imaging directions that forms a first imaging angle;
acquiring two radiation images of the subject from two imaging directions that form a second imaging angle which differs from the first imaging angle;
displaying the radiation images of the first imaging angle as two dimensional images while displaying the radiation images of the second imaging angle as a stereoscopic image;
receiving a designation of a predetermined position within the radiation images of the first imaging angle that are displayed as the two-dimensional images;
obtaining the received position information;
obtaining three-dimensional position information by projecting the received position information within the radiation images of the first imaging angle onto the radiation images imaged from the second imaging angle based on the received position information; and
displaying markers on the radiation images of the second imaging angle which are displayed as the stereoscopic image based on the obtained three-dimensional position information.

According to the radiation image display apparatus of the present invention, two radiation images are acquired by emitting radiation to a subject from two imaging directions that form a first imaging angle and are displayed as two-dimensional images, and two radiation images of two imaging directions that form a second imaging angle which differs from the first imaging angle are acquired and displayed as a stereoscopic image. Designation of a predetermined position within the radiation images of the first imaging angle which are displayed as the two-dimensional images, is received, and the received position information is projected onto the radiation images of the second imaging angle thereby obtaining three-dimensional position information. Based on the obtained three-dimension position information, three-dimensional markers are displayed on the radiation images of the second imaging angle, which are displayed as the stereoscopic image. This enables instantaneous confirmation regarding whether targeting has been performed correctly on the radiation images of the first imaging angle which are displayed as the two-dimensional images, by the three-dimensional markers on the radiation images of the second imaging angle, which are displayed as a stereoscopic image, whereby diagnostic efficiency can be improved.

### BRIEF DESCRIPTION OF THE DRAWINGS

Figure 1 is a schematic configuration diagram of a mammography display system that utilizes an embodiment of a radiation image display apparatus of the present invention.
Figure 2 illustrates an arm section of the mammography display system and displaying system shown in Figure 1 viewed from the right side in Figure 1.
Figure 3 illustrates an imaging platform of the mammography display system shown in Figure 1 viewed from above.
Figure 4 is a block diagram of the interior of a computer of the mammography display system shown in Figure 1, illustrating the schematic configuration thereof.
Figure 5 is a flowchart for explaining the operations of the mammography display system that utilizes an embodiment of the radiation image display apparatus of the present invention.
Figure 6 is a pattern diagram illustrating one example of radiation images of the first imaging angle, which are displayed as two-dimensional images by a display section.
Figure 7 is a pattern diagram illustrating one example of radiation images of the second imaging angle, which are displayed as a stereoscopic image, and a stereoscopic cursor.
Figure 8 is a block diagram of a mammography display system that utilizes another embodiment of the radiation image display apparatus of the present invention.

### DESCRIPTION OF THE PREFERRED EMBODIMENTS

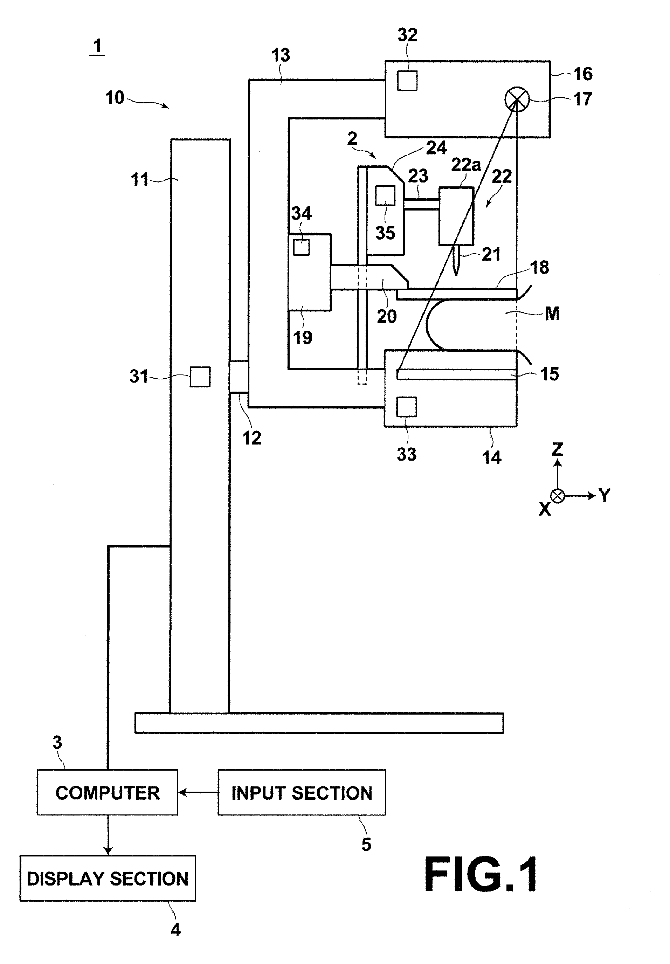

Hereinafter, a stereoscopic mammography display system that utilizes an embodiment of the radiation image display apparatus of the present invention will be described with reference to the accompanying drawings. The mammography display system of the present embodiment is a system that may function as a stereo breast biopsy apparatus by attaching a removably attachable biopsy unit thereto. First, the schematic configuration of the entire mammography display system of the present embodiment will be described. Figure 1 schematically illustrates the configuration of the mammography display system with a biopsy unit being attached thereto.

As shown in Figure 1, the mammography display system 1 of the present embodiment includes a mammography apparatus 10, a computer 3 connected to the mammography apparatus 10, and a display section 4 and an input section 5 connected to the computer 3.

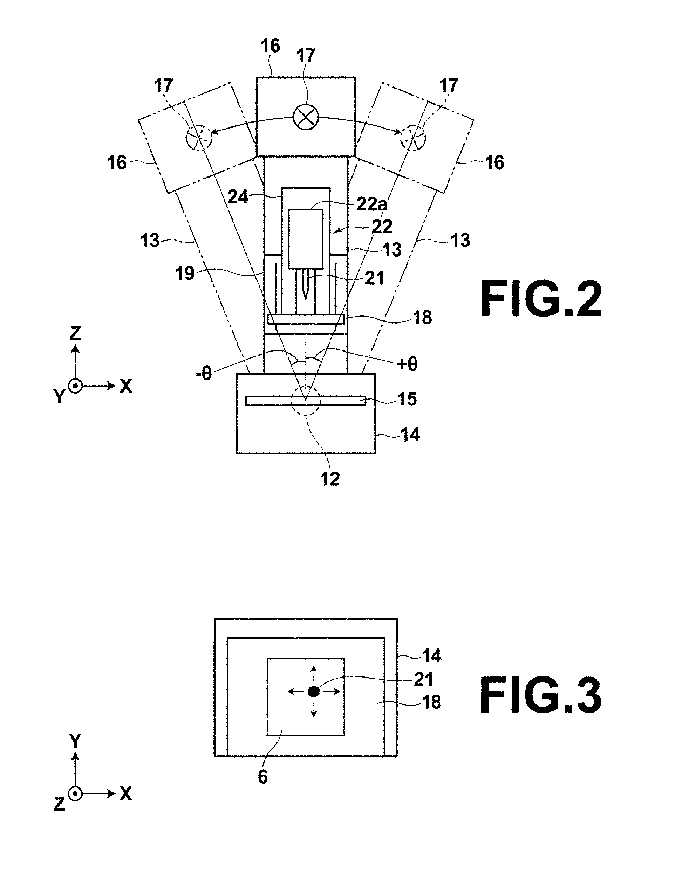

As shown in Figure 1, the mammography apparatus 10 includes a base 11, a rotary shaft 12 which is movable in the vertical direction (Z direction) with respect to the base 11, as well as being rotatable, and an arm section 13 coupled to the base 11 via the rotary shaft 12. Figure 2 shows the arm section 13 viewed from the right side of Figure 1.

The arm section 13 is in the shape of the letter C. An imaging platform 14 is attached to one side of the arm section 13 and a radiation emission section 16 is attached to the other side so as to face the imaging platform 14. The rotation and vertical movement of the arm section 13 are controlled by an arm controller 31 incorporated in the base 11.

The imaging platform 14 includes therein a radiation image detector 15, such as a flat panel detector or the like, and a detector controller 33 that controls charge signal reading from the radiation image detector 15. The imaging platform 14 further includes a circuit board having thereon a charge amplifier that converts charge signals read out from the radiation image detector 15 to voltage signals, a correlated double sampling circuit that samples the voltage signals output from the charge amplifier, and an A/D converter that converts the voltage signals to digital signals, and the like.

The imaging platform 14 is configured to be rotatable with respect to the arm section 13 and the orientation of the imaging platform 14 can be fixed with respect to the base 11 even when the arm section 13 is rotated with respect to the base 11.

The radiation image detector 15 is capable of being used repeatedly for radiation image recording and reading. As the radiation image detector 15, a so-called direct type radiation image detector that generates charges by receiving radiation directly or a so-called indirect type radiation image detector that converts radiation to visible light first and then converts the visible light to charge signals may be used. As for the radiation image signal readout method, a so-called TFT (thin film transistor) readout method in which radiation image signals are read out by switching TFT switches ON/OFF or an optical readout method in which radiation image signals are read out by directing readout light to the detector may be used. Further, as for the indirect type radiation image detector, a detector that utilizes CMOS (Complementary Metal Oxide Semiconductor) or CCD (Charge Coupled Device Image Sensor) may be applied.

The radiation emission section 16 includes therein a radiation source 17 and a radiation source controller 32. The radiation source controller 32 controls the emission timing of radiation from the radiation source 17 and radiation generation conditions (tube current, time, tube voltage, and the like) for the radiation source 17.

Further, a compression plate 18, disposed above the imaging platform 14, for holding down and compressing a breast M, a support section 20 for supporting the compression plate 18, and a moving mechanism 19 for moving the support section 20 in the vertical direction (Z direction) are provided at the center portion of the arm section 13. The position and compression pressure of the compression plate 18 are controlledby a compression plate controller 34. Figure 3 shows the compression plate 18 of Figure 1 viewed from above. As shown in Figure 3, the compression plate 18 has an opening 6 of a size of about 10×10 cm to enable biopsy to be performed with a breast being fixed by the imaging platform 14 and the compression plate 18.

The biopsy unit 2 is mechanically and electrically connected to the mammography display system 1 when the base portion thereof is inserted into the opening of the support section 20 of the compression plate 18 and the lower end of the base portion is fixed to the arm section 13.

The biopsy unit 2 has a removably attachable biopsy needle unit 22 that includes a biopsy needle 21 to be punctured into the breast, a needle support section 23 for supporting the biopsy needle unit 22, and a moving mechanism 24 for moving the biopsy needle unit 22 in the X, Y, and Z directions shown in Figures 1 to 3 by moving the needle support 23 along a rail or by extending or retracting the needle support 23. The position of the tip of the biopsy needle 21 of the biopsy needle unit 22 is recognized as position coordinates (x, y, z) in a three-dimensional space and controlled by a needle position controller 35 of the moving mechanism 24. Note that the direction perpendicular to the plane of the drawing sheet of Figure 1 is the X direction, the direction perpendicular to the plane of the drawing sheet of Figure 2 is the Y direction, and the direction perpendicular to the plane of the drawing sheet of Figure 3 is the Z direction.

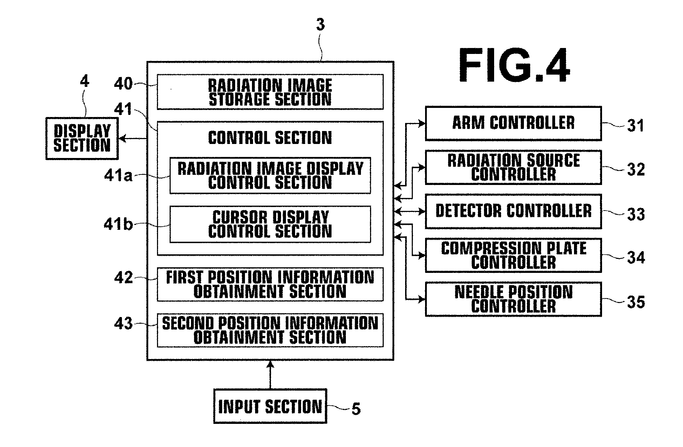

The computer 3 includes a central processing unit (CPU) and a storage device, such as a semiconductor memory, hard disk, SSD, or the like. These hardware components constitute a radiation image storage section 40, a control section 41, a first position information obtainment section 42 and a second position information obtainment section 43 as shown in Figure 4.

The radiation image storage section 40 is a section for storing radiation image signals for each imaging angle obtained by the radiation image detector 15.

The control section 41 outputs predetermined control signals to each of the controllers 31 to 35 to perform control of the entire system. The control section 41 includes a radiation image display control section 41a which displays radiation images at the display section 4 based on the radiation image signals stored in the radiation image storage section 40 and a cursor display control section 41b which displays stereoscopic cursors together with the radiation images at the display section 4.

The control section 41 of the present embodiment specifically performs control so as to capture two radiation images at a first imaging angle (±15°) to be described later and to display the radiation images as two-dimensional images at the display section 4 as well as to capture two radiation images at a second imaging angle (±2°) to be described later and display the radiation images as a stereoscopic image at the display section 4. A specific control method of the control section 4 will be described later in detail.

The position information obtainment section 42 obtains position information regarding abnormal shadows or the like designated by an observer within the radiation images captured at the first imaging angle (±15°) and displayed as the two-dimensional image, and outputs the obtained position information to the second position information obtainment section 43.

The second position information obtainment section 43 obtains three-dimensional position information by projecting the position information, designated within the radiation images captured by the first position information obtainment section 42 and displayed as the two-dimensional images, onto the two radiation images captured at the second imaging angle (±2°) and displayed as the stereoscopic image, based on the position information obtained by the first position information obtainment section 42. The three-dimensional position information obtained by the second position information obtainment section 43 is output to the cursor display control section 41b of the control section 41. The cursor display control section 41b displays a stereoscopic cursor on the stereoscopic image based on the input three-dimensional position information.

The input section 5 is constituted, for example, by a keyboard and a pointing device such as a mouse, and is configured to be able to designate the positions of abnormal shadows and the like within the radiation images displayed as the two-dimensional images on the display section 4 and the stereoscopic image displayed on the display section 4 with a cursor. The input section 5 also receives input of imaging conditions or operational instructions from the operator.

The display section 4 is designed to display a stereoscopic image using two radiation image signals output from the computer 3 and to display the radiation images based on the two radiation image signals as two-dimensional images, respectively. A configuration for displaying the stereoscopic image may include, for example, a configuration in which the radiation images based on the two radiation image signals are displayed on two different screens respectively and one of them is input to the right eye while the other is input to the left eye of an observer using a half mirror or a polarization glass. Alternatively, for example, a configuration may be employed in which a stereoscopic image is generated by displaying two radiation images shifted by a predetermined amount of parallax to be superimposed on each other and viewing the radiation images with a polarization glass. Further, a configuration may be employed in which a stereoscopic image is generated by displaying two radiation images on a 3D liquid crystal display that enables stereoscopic viewing of two radiation images as in the parallax barrier method or lenticular method. Further, a configuration in which a monitor for displaying a stereoscopic image is separately disposed from a monitor for displaying two-dimensional images or a configuration in which a single monitor is employed in the case that both types of images can be displayed on the same screen may be applied.

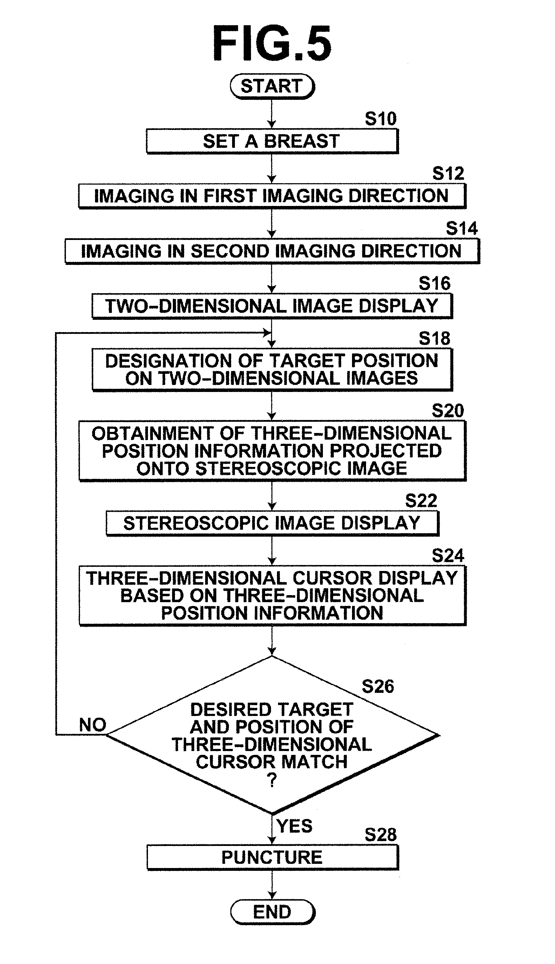

An operation of the mammography display system of the present embodiment will now be described with reference to the flowchart shown in Figure 5.

First, a breast M is placed on the imaging platform 14 and the breast is compressed by the compression plate 18 at a predetermined pressure (step S10).

Next, various imaging conditions are input from the input section 5 by the operator, and then an instruction for starting an imaging operation is input. At this time, the biopsy needle unit 22 stands by above and is not punctured into the breast yet.

Then, if an instruction to start the imaging operation is received from the input section 5, a stereoscopic image of the breast M is captured. Specifically, the control section 41 reads out a first imaging angle and a second imaging angle which have been set in advance, and outputs information regarding the readout first and second imaging angles to the arm controller 31.

The first imaging angle refers to an angle formed by two imaging directions for two radiation images which are used for targeting an abnormal shadow or the like. In the present embodiment, the first imaging angle is set to ±15° in advance, as described above. In this case, the first imaging angle is not limited to this angle, but any angle between ±10° and ±30° may be adopted, for example.

The second imaging angle refers to an angle formed by two imaging directions for two radiation images which are used for displaying a stereoscopic image. In the present embodiment, the second imaging angle is set to ±2° in advance, as described above. In this case, the second imaging angle is not limited to this angle, but any angle within a range from ±2° to ±5° may be adopted, for example. Moreover, two imaging directions that form the second imaging angle need not necessarily be symmetrical and the two imaging directions may be set to 0° and 4°, for example.

Note that it is desirable for the second imaging angle to be smaller than the first imaging angle. In this manner, by causing the second imaging angle to be relatively small, stereoscopic viewing of stereoscopic images can be facilitated, while by causing the first imaging angle to be relatively large, resolution in the Z direction of the radiation images can be increased. This can improve the accuracy of targeting.

When the arm controller 31 receives the information regarding the first and second imaging angles output from the control section 41, the arm controller 31 first outputs control signals to the arm section 13 based on the information regarding the first imaging angle (S12).

Specifically, the arm controller 31 outputs a control signal for causing the arm section 13 to rotate by +15° from the direction perpendicular to the imaging platform 14. Then, in response to this control signal, the arm section 13 rotates by +15°. Subsequently, the control section 41 outputs control signals to the radiation source controller 32 and the detector controller 33 to start emitting radiation and to start reading radiation image signals respectively. In response to these control signals, the following procedures are performed: radiation is emitted from the radiation source 17; a radiation image of a breast captured from the +15° direction is detected by the radiation image detector 15; radiation image signals are read out by the detector controller 33; the radiation image signals are subjected to predetermined signal processes; and the radiation image signals subjected to the signal processes are stored in the radiation image storage section 40 of the computer 3.

Next, the arm controller 31 outputs a control signal for causing the arm section 13 to rotate by -15° from the direction perpendicular to the imaging platform 14. Then, in response to this control signal, the arm section 13 temporarily returns to the perpendicular direction and then rotates by -15°. Subsequently, the control section 41 outputs control signals to the radiation source controller 32 and the detector controller 33 to start emitting radiation and to start reading radiation image signals respectively. In response to these control signals, the following procedures are performed: radiation is emitted from the radiation source 17; a radiation image of a breast captured from the -15° direction is detected by the radiation image detector 15; radiation image signals are read out by the detector controller 33; the radiation image signals are subjected to predetermined signal processes; and the radiation image signals subjected to the signal processes are stored in the radiation image storage section 40 of the computer 3.

As described above, when the imaging at the first imaging angle is completed, the arm controller 31 outputs a control signal to the arm section 13 based on the information regarding the second imaging angle (S14).

Specifically, the arm controller 31 outputs a control signal for causing the arm section 13 to rotate by +2° from the direction perpendicular to the imaging platform 14, and in response to this signal, the arm section 13 rotates by +2°. Subsequently, the control section 41 outputs control signals to the radiation source controller 32 and the detector controller 33 to start emitting radiation and to start reading radiation image signals respectively. In response to these control signals, the following procedures are performed: radiation is emitted from the radiation source 17; a radiation image of a breast captured from the +2° direction is detected by the radiation image detector 15; radiation image signals are read out by the detector controller 33; the radiation image signals are subj ected to predetermined signal processes; and the radiation image signals subjected to the signal processes are stored in the radiation image storage section 40 of the computer 3.

Next, the arm controller 31 outputs a control signal for causing the arm section 13 to rotate by -2° from the direction perpendicular to the imaging platform 14. Then, in response to this control signal, the arm section 13 temporarily returns to the perpendicular direction and then rotates by -2°. Subsequently, the control section 41 outputs control signals to the radiation source controller 32 and the detector controller 33 to start emitting radiation and to start reading radiation image signals respectively. In response to these control signals, the following procedures are performed: radiation is emitted from the radiation source 17; a radiation image of a breast captured from the -2° direction is detected by the radiation image detector 15; radiation image signals are read out by the detector controller 33; the radiation image signals are subjected to predetermined signal processes; and the radiation image signals subjected to the signal processes are stored in the radiation image storage section 40 of the computer 3.

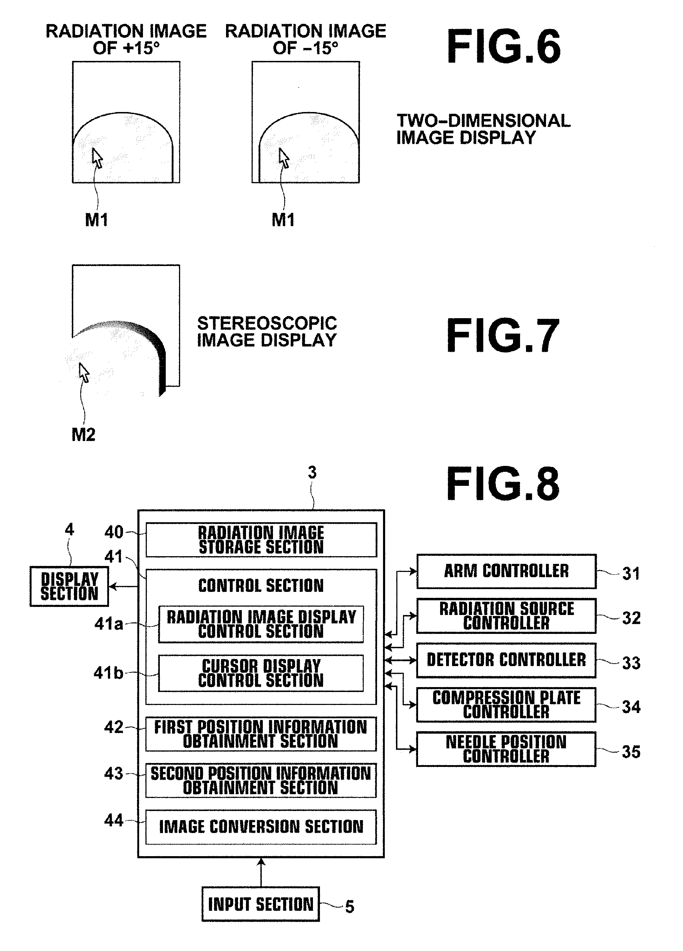

When the radiation images are captured at the first and second imaging angles, two radiation image signals obtained at the first imaging angle are read out by the radiation image display control section 41a from the radiation image storage section 40, the radiation image signals are subjected to predetermined signal processes, and the radiation image signals subjected to the signal processes are output to the display section 4. Then, both the radiation image of +15° and the radiation image of -15° are displayed as two-dimensional images at the display section 4 based on the two radiation image signals obtained at the first imaging angle (S16). Figure 6 schematically illustrates a diagram of two-dimensional image display.

Next, when two-dimensional images of a breast are displayed in the manner described above, a calcification, tumor, or the like in the breast are found by the observer. Subsequently, in the case that these tissues are desired to be removed by the biopsy unit 2, the observer designates a target such as a calcification, tumor, or the like in each of the radiation image of +15° and the radiation image of -15° displayed at the display section 4 (S18).

Designation of the target may be performed by using a pointing device such as a mouse or the like in the input section 5, for example. Specifically, as shown in Figure 6, a cursor M1 for targeting is displayed, and then the observer may move this cursor M1 to a desired target position by using the input section 5 so as to designate the target.

Position information of the cursor M1 designated by the observer on the two radiation images are obtained by the first position information obtainment section 42, respectively. Then, the first position information obtainment section 42 outputs the position information of the cursor M1 on the two radiation images to the second position information obtainment section 43.

The second position information obtainment section 43 obtains three-dimensional position information by projecting the two pieces of position information onto the two radiation images for the stereoscopic image display, which have been captured at the second imaging angle (±2°), based on the position information of the target on the input two radiation images (S20) . Then, the three-dimensional position information obtained by the second position information obtainment section 43 is output to the cursor display control section 41b.

In this case, this three-dimensional position information can be calculated by using the trigonometry based on the position information of the target on the two radiation images, the two imaging directions that form the first imaging angle, the two imaging directions that form the second imaging angle, and the distance between the radiation source 17 and the radiation image detector 15.

Next, the observer inputs an instruction for stereoscopic image display by using the input section 5. Then, in response to the input instruction, two radiation image signals obtained at the second imaging angle are read out from the radiation image storage section 40 by the radiation image display control section 41a, are subjected to a predetermined signal processes, and output to the display section 4. The radiation image for the right eye and the radiation image for the left eye are displayed at the display section 4 based on the two radiation image signals obtained at the second imaging angle so that a stereoscopic image is displayed at the display section 4 (S22). Figure 7 schematically illustrates a diagram of the stereoscopic image display. At this time, the stereoscopic image may be simultaneously displayed together with the two radiation images which had been previously two-dimensionally displayed, or may be displayed by switching from the two-dimensionally displayed radiation images.

Further, the cursor display control section 41b generates a cursor image signal for the right eye and a cursor image signal for the left eye that have an amount of parallax corresponding to the input three-dimensional position information based on the three-dimensional position information, and these cursor image signals are output to the display section 4. The display section 4 displays a three-dimensional cursor M2 (refer to Figure 7) having an amount of depth on the stereoscopic image (S24). In this case, the three-dimensional cursor M2 may be displayed as a color image that differs from a black-and-white stereoscopic image, or may be displayed while blinking. Alternatively, a display mode and a hidden mode may be switched according to a predetermined instruction input from the input section 5.

Then, the observer confirms whether the three-dimensional cursor M2 displayed on the stereoscopic image as described above is appropriately displayed at the position of a desired target within the stereoscopic image.

At this time, in the case that the three-dimensional cursor M2 is not appropriately displayed at the position of a desired target within the stereoscopic image (S26, No), the process returns to S18 so that targeting is performed again on the radiation images of ±15° captured at the first imaging angle. The processes S18 through S24 are repeated until the three-dimensional cursor M2 is displayed at the position of a desired target within the stereoscopic image.

In the case that the three-dimensional cursor M2 is displayed at the position of a desired target within the stereoscopic image, the observer inputs a signal indicating this fact by using the input section 5. Then, the control section 41 performs the following procedures when the signal input is performed: the position information obtained by the first position information obtainment section 42 and the three-dimensional position information obtained by the second position information obtainment section 43 are obtained; position information (x1, y1, z1) of a target on the stereoscopic image is obtained based on the obtained position information; and the obtained position information (x1, y1, z1) of the target is output to a needle position controller 35 of the biopsy unit 2.

If a predetermined operation button is pressed at the input section 5 in this state, the control section 41 outputs a control signal for moving the biopsy needle 21 to the needle position controller 35. The needle position controller 35 moves the biopsy needle 21 so that the tip of the biopsy needle 21 is disposed at the position indicated by the coordinates (x1, y1, z1+α) based on the position information (x1, y1, z1) which have been previously input. In this case, "α" refers to a value which is sufficiently large for the biopsy needle not to be punctured into the breast. Accordingly, the biopsy needle 21 is set above the target.

Thereafter, when the observer inputs an instruction for puncture of the biopsy needle 21 at the input section 5, the biopsy needle 21 is moved so that the tip of the biopsy needle 21 is disposed at the position indicated by the coordinates (x1, y1, z1) and the biopsy needle 21 is punctured into the breast under the control of the control section 41 and the needle position controller 35 (S28).

In the mammography display system of the above embodiment, a total of four radiation images are captured at two imaging directions that form the first imaging angle and at two imaging directions that form the second imaging direction. However, the radiation images of the two imaging directions that form the second imaging angle need not necessarily be captured and two radiation images of two imaging directions that form the second imaging angle may be generated by using the two radiation images captured from the two imaging directions that form the first imaging angle instead.

Specifically, for example, as shown in Figure 8, an image conversion section 44 is provided in the computer 3 and the image conversion section 44 may generate the radiation image of +2° and the radiation image of -2° by using the radiation image of +15° and the radiation image of -15°. A method for generating the radiation images of ±2° from the radiation images of +15° may include: for example, a method in which the radiation images of ±15° are subjected to a process that reduces the images only by 1/7.5 in the X direction; a method in which objects such as mammary glands, a calcification, a tumor, or the like contained within the radiation images of +15° are shifted only by a shift amount corresponding to a position in the X direction therefrom; or a method in which the amount of parallax between the two images is reduced by shifting the radiation images of ±15° as a whole. In short, any process for approximating stereoscopic effects when displaying the radiation images of ±2° as a stereoscopic image may be employed.

As described above, radiation images of two imaging directions that form the second imaging angle are generated by using two radiation images imaged from two imaging directions that form the first imaging angle, which enables reduction of the dose of radiation received by a subject.

Although the above embodiments were described as cases in which an embodiment of the radiation image display system of the present invention is applied to a mammography display system, the subject of the present invention is not limited to breasts and the present invention may also be applied to radiation image display systems that capture chest regions or head regions.

## Claims

1. A radiation image display apparatus comprising:
a radiation image acquisition section which acquires two radiation images imaged by emitting radiation to a subject from two imaging directions that form a first imaging angle and which acquires two radiation images of the subject from two imaging directions that form a second imaging angle which differs from the first imaging angle;
a display section which displays the radiation images of the first imaging angle as two dimensional images and which displays the radiation images of the second imaging angle a stereoscopic image;
a position designation receiving section which receives designation of a predetermined position within the radiation images of the first imaging angle which are displayed as the two-dimensional images;
a first position information obtainment section which obtains position information received by the position designation receiving section; and
a second position information obtainment section which obtains three-dimensional position information by projecting the position information within the radiation images of the first imaging angle, that has been obtained by the first position information obtainment section, onto the radiation images of the second imaging angle based on the obtained position information; wherein
the display section displays three-dimensional markers on the radiation images of the second imaging angle, which are displayed as the stereoscopic image, based on the three-dimensional position information obtained by the second position information obtainment section.

2. The radiation image display apparatus as claimed in claim 1, wherein the first imaging angle is larger than the second imaging angle.

3. The radiation image display apparatus as claimed in claim 1 or 2, wherein the radiation images of the second imaging angle are captured by emitting radiation to the subj ect from the two imaging directions that form the second imaging angle.

4. The radiation image display apparatus as claimed in claim 1 or 2, further comprising an image conversion section which generates radiation images of the second imaging angle by using the radiation images of the first imaging angle; and wherein
the radiation image acquisition section acquires radiation images of the second imaging angle generated by the image conversion section.

5. A radiation image display method comprising the steps of:
acquiring two radiation images imaged by emitting radiation to a subject from two imaging directions that forms a first imaging angle;
acquiring two radiation images of the subject from two imaging directions that form a second imaging angle which differs from the first imaging angle;
displaying the radiation images of the first imaging angle as two dimensional images while displaying the radiation images of the second imaging angle as a stereoscopic image;
receiving a designation of a predetermined position within the radiation images of the first imaging angle that are displayed as the two-dimensional images;
obtaining the received position information;
obtaining three-dimensional position information by projecting the received position information within the radiation images of the first imaging angle onto the radiation images imaged from the second imaging angle based on the received position information; and
displaying markers on the radiation images of the second imaging angle which are displayed as the stereoscopic image based on the obtained three-dimensional position information.
